# EUROPEAN PATENT APPLICATION

(11) **EP 1 736 135 A1**
(43) Date of publication of application: **27.12.2006**
(21) Application number: 06253193.4
(22) Date of filing: 21.06.2006
(51) Int. Cl.: A61K 8/02, A61K 8/24, A61K 8/19, A61K 8/22, A61K 8/23, A61Q 11/00

(54) **Oral care compositions, devices, and methods of using the same**

(30) Priority: 22.06.2005 US 158600; 01.08.2005 US 195448
(71) Applicant: McNeil-PPC, Inc., Skillman, NJ 08558-9418 (US)
(72) Inventor: Sharma, Deepak, Plainsboro NJ 08536 (US); Tyndall, David, Medway MA 02053 (US); Passi, Rajeev Kumar, Marlborough MA 01752 (US)
(74) Representative: Mercer, Christopher Paul

(57) **Abstract**

Oral care compositions, devices and methods are disclosed that employ an amorphous calcium phosphate or a precursor or derivative thereof, and a tooth whitening agent.

## Description

### CROSS REFERENCE TO RELATED APPLICATIONS

This application is a continuation, and claims the benefit of priority from U.S. Patent Application Serial No. 11/158,600, filed June 22, 2005, the contents of which is hereby incorporated by reference herein in its entirety.

### TECHNICAL FIELD

This invention relates to oral care compositions, and to devices and methods that employ the same.

### BACKGROUND

Dental caries is initiated by the demineralization of hard tissue on teeth by organic acids. A substantial number of mineral ions can be removed from the hydroxyapatite (HAP) lattice network of tooth enamel without destroying its structural integrity; however, such demineralized enamel leads to the formation of tiny lesions that transmit hot, cold, pressure and pain more readily than normal enamel, and such lesions are thought to be precursors to dental caries formation. In some instances, frequent use of peroxide containing products, e.g., tooth whitening products, has contributed to demineralization and hypersensitivity.

Recently, amorphous calcium phosphate (ACP) and its fluoride (ACPF) and carbonate-containing (ACCP) derivatives have been used to remineralize and to desensitize teeth. ACP, when exposed and/or dissolved in water, rapidly reverts to a more thermodynamically stable form, e.g., hydroxyapatite (HAP), which is insoluble or nearly so, and, thus, calcium and phosphate ions are not biologically available. Some have solved this reversion problem by providing either two part or dual delivery systems.

Remineralization of teeth has been discussed in U.S. Patents Nos. 5,037,639, 5,145,668, 5,268,167, 5,437,587, 5,427,768, 5,460,803, 5,476,647, 5,508,342, 5,534,244, 5,562,895, 5,891,448, 5,833,954, 5,981,475, 5,993,786,6,000,341,6,056,930, 6,214,368, 6,521,215, and 6,780,844; and whitening teeth has been discussed in U.S. Patent Nos. 6,682,721, 6,689,344, 6,780,401, 6,893,629, 6,551,579, 6,136,297, 6,096,328, 6,045,811, 5,989,569, 5,894,017, 5,891,453, and 5,879,691, the entire contents ofeach patent of this paragraph is hereby incorporated by reference herein in its entirety.

### SUMMARY

Generally, the invention relates to oral care devices and compositions that can, e.g., simultaneously remineralize and whiten the teeth, reduce sensitivity and/or harden teeth. In some embodiments, the devices and compositions are maintained under anhydrous conditions until used in the oral cavity to reduce reversion of the amorphous calcium phosphate to an insoluble form and/or to maintain the potency of the whitening agent.

In one aspect, the invention features an oral care device, e.g., a strip or a mouthpiece, configured to contact one or more teeth. The oral care device includes an amorphous calcium phosphate or a precursor or derivative thereof, and a tooth whitening agent.

In some embodiments, the amorphous calcium phosphate includes a stabilized amorphous calcium phosphate, e.g., an amorphous calcium phosphate that includes a metal or a metalloid. Metals include, e.g., zirconium, silicon, titanium, or mixtures thereof. Specific examples of stabilized amorphous calcium phosphates include zirconium-modified amorphous calcium phosphate (Zr-ACP), titanium-modified amorphous calcium phosphate (Ti-ACP), silicon-modified amorphous calcium phosphate (Si-ACP), amorphous calcium phosphate fluoride (ACPF), amorphous calcium carbonate phosphate (ACCP) or mixtures of these. The stabilized amorphous calcium phosphate can include, e.g., an amino acid, a polypeptide or derivatives of an amino acid or a polypeptide. In a specific embodiment, the polypeptide includes a casein phosphopeptide, or a derivative thereof. A synthetic polymer, e.g., can also be used to stabilize the amorphous calcium phosphate.

The tooth whitening agent can be, e.g., a peroxides, a metal chlorite, a perborate, a percarbonate, a peroxyacid, a persulfate, a carbamide, a hypochlorite, chlorine dioxide, or mixtures of these. In specific embodiments, the tooth whitening agent includes hydrogen peroxide, a complex of thereof, or a source thereof.

The oral care device can be, e.g., in the form of a strip. The strip can include, e.g., a polymeric material. The polymeric material can be, e.g., polyvinylpyrrolidone, a cellulosic polymer, polyvinyl alcohol, hydroxyethyl cellulose, hydroxypropyl cellulose, hydroxypropylmethyl cellulose, sodium carboxymethyl cellulose, polyethylene oxide, polyethylene glycol, polyacrylic acid, silicone, hydroxypropylmethyl-cellulose phthalate (HPMCP), polyvinyl acetate, cellulose acetate phthalate, gelatin sodium alginate, carbomer, polyvinyl pyrrolidone-vinyl acetate copolymer, polyalkylvinylether-maleic acid copolymer, polypropylene glycol, polyethylene glycol-polypropylene glycol copolymers, quaternized copolymers, quaternized copolymers of vinylpyrrolidone and dimethylaminoethyl methacrylate, or mixtures thereof. In some embodiments, the polymeric material is soluble or swollen by water. The polymeric can have a flexural modulus of less than 100,000 psi, e.g., less than 25,000 psi. The polymeric material can have, e.g., a hardness of less than 100 Shore A, e.g., less than 50 Shore A.

In some embodiments, the strip has more than a single layer.

In a specific embodiment, the strip includes a first layer configured to contact one or more teeth that includes the amorphous calcium phosphate; a third layer; and a second layer sandwiched between the first and third layers, the second layer including the whitening agent. The first layer can include, e.g., polyvinylpyrrolidone, the second layer can include, e.g., polyvinylpyrrolidone, and the third layer can include a barrier material, e.g., hydroxypropylmethylcellulose phthalate (HPMCP).

In some embodiments, the layers are each formed by casting.

In another aspect, the invention features a method of treating teeth. The method includes contacting one or more teeth with a device that includes an amorphous calcium phosphate or a precursor or derivative thereof, and a tooth whitening agent. Contact is maintained with the one or more teeth for a sufficient time to at least whiten the one or more teeth. The method can used to, e.g., simultaneously remineralize and whiten teeth, treat tooth sensitivity, harden teeth and/or reduce dental lesions and/or caries.

In another aspect, the invention features an oral care composition, e.g., a liquid, a gel, a dentifrice and the like, that includes an amorphous calcium phosphate or a precursor or derivative thereof, and a tooth whitening agent.

Any of the amorphous calcium phosphates and any of the whitening agents described above can be used in any of the compositions, e.g., in the form of a liquid, gel, paste, dentifrice, oral rinse, paint or varnish.

In addition, any of the flavoring agents, a fragrances, sweeteners, coloring agents, peroxide activators, enzymes, malodor controlling agents, cleaning agents, antibacterial agents, antigingivitis agents, anti-caries agents, antiperiodontitis agents and tooth sensitivity agents can be used in any of the compositions.

In another aspect, the invention features a method of treating teeth. The method includes applying to a tooth, an oral care composition that includes an amorphous calcium phosphate or a precursor or derivative thereof, and a whitening agent. The oral care composition is allowed to maintain contact with the tooth for sufficient time to at least whiten the tooth. In some embodiments, sufficient time is less than 180 minutes, e.g., less than 120 minutes, less than 60 minutes, less than 30 minutes, less than 25 minutes, less than 20 minutes, less than 10 minutes or even less than 5 minutes. The method can be used to, e.g., simultaneously remineralize and whiten teeth, treat tooth sensitivity, harden teeth and/or reduce dental lesions and/or caries. To obtain the desired whitening results, the method can be repeated, e.g., 7-14 times, or more, e.g., 20-30 times, for 30-45 minutes each time. The method can reduce sensitivity and at the same time whiten teeth.

In another aspect, the invention features a method of simultaneously whitening and remineralizing teeth with reduced tooth sensitivity. The method includes contacting one or more teeth with a device that includes an amorphous calcium phosphate or a precursor or derivative thereof, and a tooth whitening agent. Contact is maintained with the one or more teeth for a sufficient time to at least whiten and partially remineralize the one or more teeth.

For the purposes of this disclosure, a precursor to an amorphous calcium phosphate is a compound or compounds that generate an amorphous calcium phosphate when placed in the oral cavity. Examples include calcium nitrate, calcium chloride, calcium hydroxide, calcium peroxide, calcium carbonate, potassium phosphate, dipotassium hydrogen phosphate, potassium dihydrogen phosphate, sodium phosphate, disodium hydrogen phosphate, sodium dihydrogen phosphate, or mixtures of these materials.

For the purposes of this disclosure, a derivative of an amorphous calcium phosphate is a compound that is formed from a reaction of an amorphous calcium phosphate. Examples include amorphous calcium phosphate fluoride (ACPF), amorphous calcium carbonate phosphate (ACCP), amorphous calcium carbonate phosphate fluoride (ACCPF), amorphous strontium phosphate (ASP), amorphous strontium calcium phosphate (ASCP), amorphous strontium calcium phosphate (ASCP), amorphous strontium calcium carbonate phosphate (ASCCP), and amorphous strontium calcium phosphate fluoride (ASCPF).

For the purposes of this disclosure, strip-form includes films, patches and/or wraps that are suitable to place on a tooth.

Embodiments may have one or more of the following advantages. The compositions, devices and methods allow a user to carry out tooth remineralization simultaneously with tooth whitening. The devices reduce irritation and/or burning of gums and/or sensitive cheek tissues opposite tooth surfaces. The compositions allow for a high bio-availability of calcium and phosphate ions, leading to a particularly efficient remineralization. The compositions can harden teeth, and can reduce tooth sensitivity and/or reduce dental lesions and/or dental caries. Whitening can be rapid, occurring in less than 120 minutes, e.g., less than 90 minutes, less than 60 minutes, less than 45 minutes, less than 30 minutes, less than 15 minutes, or even less than 5 minutes.

All publications, patent applications, patents, and other references mentioned herein are incorporated by reference in their entirety.

Other aspects, features, and advantages will be apparent from the description and drawings, and from the claims.

### DESCRIPTION OF DRAWINGS

Fig. 1 is a perspective, exploded view of a three-layer dental strip.
Fig. 1A is a cross-sectional view of the dental strip of Fig. 1, taken along 1A-1A.
Fig. 2 is a cross-sectional view of dental strip having two layers.
Fig. 3 is a cross-sectional view of a dental strip having a single layer.

### DETAILED DESCRIPTION

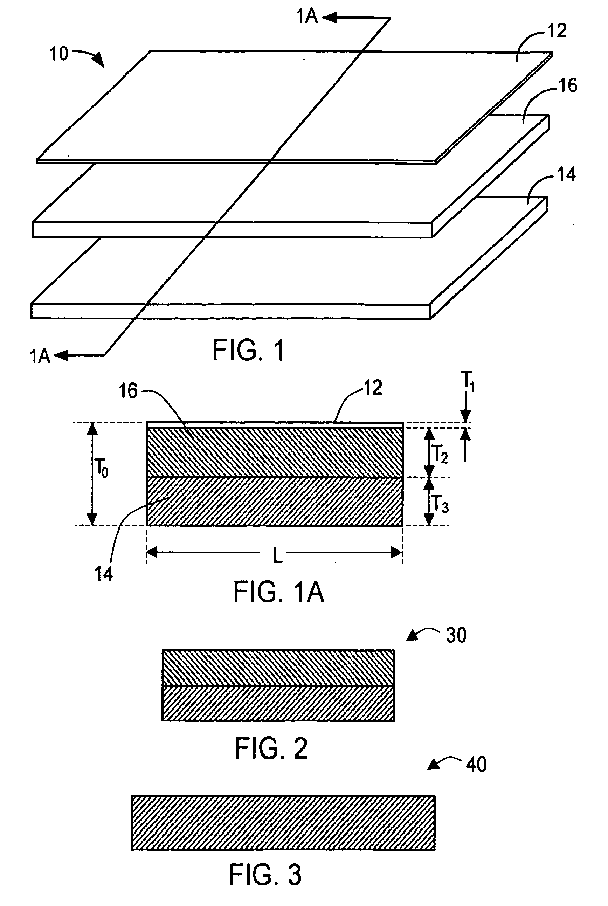

Referring to Figs. 1 and 1A, a flexible dental strip 10 formed from a polymeric material includes a first layer 12, configured to contact one or more teeth, that includes an amorphous calcium phosphate, a third layer 14, configured to contact sensitive cheek tissues opposite tooth surfaces, and a second layer 16 sandwiched between the first and third layers 12 and 14. The second layer includes the whitening agent. In some embodiments, third layer 14 provides barrier properties for unidirectional flow of actives toward tooth surfaces, thus minimizing irritation to cheeks opposite tooth surfaces. It can be advantageous, in some embodiments, to keep the whitening agent in a different layer than the amorphous calcium phosphate. Such cases include those in which the amorphous calcium phosphate reacts with the whitening agent. Generally, when making the strip, water is avoided, and the strip is maintained under anhydrous conditions until use to reduce reversion of the amorphous calcium phosphate to an insoluble form and/or to maintain the potency of the whitening agent.

In some embodiments, the flexible dental strip 10 is non-dissolvable and adherent to the teeth. For example, it is advantageous for the strip to adhere to the teeth, e.g., for a time of between about 1 minute and 8 hours, e.g., between 15 minutes and 4 hours, or between 30 minutes and 2 hours.

The amount of an amorphous calcium phosphate or a precursor or derivative thereof in the strip generally ranges from about 0.5 percent by weight to about 40 percent by weight of the strip, e.g., between about 10 percent by weight to about 35 percent by weight, or between about 10 percent by weight and 25 percent by weight. The amount of whitening agent or a precursor thereof in the strip generally ranges from about 0.5 percent by weight to about 25 percent by weight, e.g., between about 7.5 percent by weight to about 20 percent by weight.

The amorphous calcium phosphate can include a stabilized amorphous calcium phosphate, e.g., a metal- or metalloid-stabilized calcium phosphate. Examples of metal-or metalloid-stabilized are those amorphous calcium phosphates that include the elements of, e.g., zirconium, silicon, titanium, or mixtures of these. Specific examples of metal- or metalloid-stabilized calcium phosphates include zirconium-modified amorphous calcium phosphate (Zr-ACP), titanium-modified amorphous calcium phosphate (Ti-ACP), silicon-modified amorphous calcium phosphate (Si-ACP), amorphous calcium phosphate fluoride (ACPF) and amorphous calcium carbonate phosphate (ACCP). Magnesium and other divalent cations can also be used to enhance stability of ACP and its derivatives.

The stabilized amorphous calcium phosphate can also include a polypeptide, e.g., a casein phosphopeptide, or a derivative the casein phosphopeptide. In other embodiments, the amorphous calcium phosphate is, e.g., stabilized with a synthetic oligomer or polymer, an anion, a cation, polyphosphate, a pyrophosphate, an amino acid, or compatible mixtures of any of these.

Stabilized amorphous calcium phosphates are commercially available from Hawk Creek Laboratory, Inc, Hanover, PA.

The tooth whitening agent can be, e.g., a peroxide, a metal chlorite, a perborate, a percarbonate, a peroxyacid, a persulfate, a carbamide, a hypochlorite, chlorine dioxide, a precursor to any of the just described whitening agents, or compatible mixtures of any of these materials. For example, the tooth whitening agent can include hydrogen peroxide, a complex thereof, e.g., a polymer adduct of hydrogen peroxide, or a source thereof.

In some embodiments, the strip is formed from a polymeric material. Examples include polyvinylpyrrolidone, a cellulosic polymer, polyvinyl alcohol, hydroxyethyl cellulose, hydroxypropyl cellulose, hydroxypropylmethyl cellulose, sodium carboxymethyl cellulose, polyethylene oxide, polyethylene glycol, polyacrylic acid, silicone, hydroxypropylmethyl-cellulose phthalate (HPMCP), polyvinyl acetate, cellulose acetate phthalate, gelatin sodium alginate, carbomer, polyvinyl pyrrolidone-vinyl acetate copolymer, polyalkylvinylether-maleic acid copolymer, polypropylene glycol, polyethylene glycol-polypropylene glycol copolymers, quaternized copolymers, quaternized copolymer of vinylpyrrolidone and dimethylaminoethyl methacrylate, or mixtures of these polymeric materials. In some embodiments, the polymeric material is soluble or swollen by water.

In embodiments when it is desired not to have a layer swell or dissolve in the oral cavity, non-swellable or non-dissolvable materials, e.g., polymers, are selected for the layer. Examples of such materials include hydroxypropyl methyl cellulose derivatives and copolymers, e.g., the phthalate, succinate or acetates, and silicone waxes and polymers.

In some embodiments, it is advantageous that the strip be flexible so that it can conform to the many complicated and uneven surfaces of the teeth. This can be accomplished, e.g., by using a polymer that has a room temperature flexural modulus less than 100,000 psi, e.g., less than 50,000 psi or less than 25,000 psi, as measured using a dynamic mechanical analyzer. Flexibility can also be imparted by using a polymeric material having a Shore A hardness of less than 100 Shore A, e.g., less than 75 Shore A or less than 50 Shore A. Additional flexibility can be imparted by adding a plasticizer. The plasticizer can include, e.g., between about 0.1 % and about 30% by weight of the strip. Specific examples of plasticizers include polyols, such as polyethylene glycol, triethyl citrate, propylene glycol, glycerin, sorbitol, glycerol acetate or mixtures of these plasticizers.

In some embodiments, dental strip 10 can include a flavoring agent, a fragrance and/or a sweetener. Flavoring agents include, e.g., natural or synthetic essential oils, as well as various flavoring aldehydes, esters, alcohols, and other materials. Examples of essential oils include oils of spearmint, peppermint, wintergreen, sassafras, clove, sage, eucalyptus, marjoram, cinnamon, lemon, lime, grapefruit, and orange The flavoring agent, fragrance and/or sweetener can be incorporated into the strip composition at a concentration, e.g., of between about 0.1 % and about 10% by weight, e.g., between about 0.1 % to about 2 % by weight.

Coloring agents, e.g., pigments and dyes, can be included in the strips. For example, in some instances, it is desirable to have a colored strip that provides a visual cue to the user that it is time to remove the strip. The colored strip can be used in combination with a chemical or bio-sensor.

In some embodiments, the tooth whitening agent and the strip further includes a peroxide activator. Peroxide activators can increase the bleaching efficacy of peroxide components. Examples of peroxide activators include metals, e.g., iron, copper, or manganese, pH modifiers, e.g., hydroxide salts, zeolites, and photosensitizers, e.g., titanium dioxide.

The strip can also include ingredients such as enzymes, malodor controlling agents, cleaning agents, such as phosphates, antibacterial agents, antigingivitis agents, anticarries agents, antiperiodontitis agents and tooth sensitivity agents. Examples of agents include cetylpyridinium chloride, chlorhexidine, zinc chloride and potassium nitrate.

In some embodiments, each layer 12, 14 and 16 of strip 10 is formed from a different polymeric material.

The strip can be formed by casting the polymeric layers. The strip can also be formed by extrusion, e.g., co-extrusion, or molding, e.g., co-molding.

The rate at which the whitening agent and/or amorphous calcium phosphate is solubilized (in the case of a solid whitening agent), and subsequently released to a tooth surface can be controlled by properties such as, but not limited to, the film thickness, polymer properties, e.g., structure, molecular weight, type, hardness and flexural modulus, properties of the whitening agent, properties of the amorphous calcium phosphate, and the concentration of the whitening agent and amorphous calcium phosphate.

The thicknesses of the film layer may affect the residence time of any ingredients in the strip, including residence time of the whitening agent and amorphous calcium phosphate. The residence time can, for example, be determined by a combination of the composition of the polymer and the thickness of the film.

In some embodiments, it is advantageous to minimize the overall thickness of the strip since overall thickness can influence the mouth feel of the product, which can relate to user acceptance and compliance. Additionally, the thickness of the film can also affect its ability to conform and adhere to teeth in an efficient manner.

Referring particularly to Fig. 1A, an overall thickness To of the strip is, e.g., from about 0.01 mm to about 2 mm, e.g., from about 0.2 mm to about 1.5 mm; a thickness T₁ of the first layer 12 is, e.g., from about 0.01 mm to about 0.5 mm, e.g., from about 0.02 mm to about 0.3 mm; a thickness T₂ of the second layer 16 is, e.g., from about 0.05 mm to about 1 mm, e.g., from about 0.1 mm to about 0.8 mm; a thickness T₃ of the third layer 14 is, e.g., from about 0.05 mm to about 1 mm, e.g., from about 0.1 mm to about 0.8 mm; and a length of the strip L is from about 4 mm to about 40 mm, e.g., from about 10 mm to about 35 mm, or from about 10 mm to about 25 mm.

In a specific embodiment, the first and second layers 12 and 14 are cast from polyvinylpyrrolidone, and the third layer 16 is formed from hydroxypropylmethyl-cellulose phthalate (HPMCP) having a molecular weight that is greater than 100,000, as measured relative to mono-disperse polyethylene glycol standards in tetrahydrofuran as solvent. This provides for a nearly impermeable third layer 14 and substantially prevents the whitening agent from reaching the sensitive cheek tissues that are opposite the tooth surfaces.

Strip 10 can be used, e.g., to simultaneously remineralize and whiten teeth. In use, strip 10 is oriented such that layer 12 contacts the teeth of the user. Layer 14 then protects gums and sensitive cheek tissues from being exposed to the whitening agent that is in layer 16. After contact is made, the amorphous calcium phosphate diffuses from strip 10, as does the whitening agent, toward the tooth surfaces. Contact with the teeth is maintained until the desired whitening result is obtained. It may be necessary to repeat the steps with a fresh strip 10 to obtain the desired whitening effect. In some embodiments, sufficient time is less than 30 minutes, e.g., less than 15 minutes, 10 minutes or less than 5 minutes. Effective remineralization is also achieved using this composition/device containing ACP and a hydrogen peroxide source. Without wishing to bound by any particular theory, it is believed that the hydrogen peroxide molecules create a microenvironment that mimics the "natural" carbonic acid chemistry, and facilitates remineralization using soluble Ca²⁺ and PO₄³⁻ ions. It is believed that delivering Ca²⁺ and PO₄³⁻ ions in this manner can remineralize the enamel and whiten teeth.

Oral care compositions, e.g., in the form of a liquid, gel, paste, dentifrice, oral rinse, paint or varnish, that include an amorphous calcium phosphate or a precursor or derivative thereof, and a tooth whitening agent can also be used, e.g., to simultaneously remineralize and whiten the teeth. Generally, when making the compositions, water is avoided, and the compositions are maintained under anhydrous conditions until use to reduce reversion of the amorphous calcium phosphate to an insoluble form and/or to maintain the potency of the whitening agent. In some embodiments, the initial total amount of water in composition is less than 1 percent by weight, e.g., less than 0.5 percent by weight, less than 0.25 percent by weight, less than 0.1 percent by weight, or less than 0.05 percent by weight.

Any of the amorphous calcium phosphates and any of the whitening agents described above can be used in any of the compositions, e.g., in the form of a liquid, gel, paste, dentifrice, oral rinse, paint or varnish.

In addition, any of the flavoring agents, a fragrances, sweeteners, coloring agents, peroxide activators, enzymes, malodor controlling agents, cleaning agents, antibacterial agents, antigingivitis agents, anticarries agents, antiperiodontitis agents and tooth sensitivity agents can be used in any of the compositions.

The amount of an amorphous calcium phosphate or a precursor or derivative thereof in the compositions depends upon the form used, but the amount generally ranges from about 5 percent by weight to about 30 percent by weight, e.g., 7.5 percent to about 25 percent by weight, or 10 percent to about 20 percent by weight. The amount of whitening agent in the compositions depends upon the form used, but the amount generally ranges from about 1 percent by weight to about 20 percent by weight, e.g., 3 percent to about 15 percent by weight, or 5 percent to about 10 percent by weight.

The oral care composition can further include a polymeric material. For example, the polymeric material can be a soluble or swellable polymeric material such that a thick gel results.

The oral care composition can further include a solvent, e.g., a volatile or a nonvolatile solvent. Generally, volatile solvents are those having a normal atmospheric boiling point of less than 100 °C. Examples of volatile solvents include ethanol, propanol, isopropanol, tetrahydrofuran, or mixtures of these solvents. Examples of nonvolatile solvents include polyols, glycols, glycerol, propylene glycol, or mixtures of these solvents.

An oral care composition in the form of an anhydrous liquid can be applied, e.g., using a small wiper brush. In some embodiments, the oral care composition includes a polymeric material, e.g., a cellulosic material that upon application precipitates out of the liquid composition, providing an film on teeth to which it has been applied. This can, e.g., provide a visual cue to the user that indicates that the product is both present on the teeth, and is working to whiten and remineralize the teeth.

In use, any of the oral care compositions described above can be used to, e.g., simultaneously remineralize and whiten teeth. Generally, the oral care composition is applied to a tooth or teeth, and allowed stay on the tooth or teeth for a sufficient time to at least whiten the tooth. Contact with the tooth or teeth is maintained until the desired whitening result is obtained. It may be necessary to repeat the steps with fresh composition to obtain the desired whitening effect. In some embodiments, sufficient time is less than 120 minutes, e.g., less than 90 minutes, less than 45 minutes, less than 30 minutes, less than 15 minutes, 10 minutes or even less than 5 minutes.

The composition can be removed, e.g., by rinsing or brushing. Alternatively, when the composition includes a water soluble polymeric material, the composition, in time, will simply dissolve away.

### EXAMPLES

Polyvinylpyrrolidone (PVP) having a molecular weight of about 90,000 and polyvinylpyrrolidone (PVP)-hydrogen peroxide complex (Peroxydone K 90) were both obtained from ISP, Wayne, New Jersey; amorphous calcium phosphates were obtained from Hawk Creek Laboratory, Inc, Hanover, PA; Hydroxypropylmethylcellulose phthalate (HPMCP) having a molecular weight of about 62,000 was obtained from Shin Etsu (HP-55S); and Carbopol, grade 940 NF or grade EDT 20/20, was obtained from Noveon. Each was used as received, without additional purification.

### Example 1: Three Layer Oral Care Strip / ACP and Whitening Agent in Separate Layers

The first layer (the adherent layer) was cast from the following formulation:

| | | |
|---|---|---|
| | **Ingredient** | **w/w %** |
| 1 | Ethanol (200 proof) | 94.20 |
| 2 | PVP K-90 | 5.00 |
| 3 | PEG-400 | 0.50 |
| 4 | Menthol | 0.15 |
| 5 | Sodium Saccharin | 0.15 |
| 6 | Si-ACP | 1.00 |
| | Total | 100.00 |

The second layer (middle layer) was cast from the following formulation:

| | **Ingredient** | **w/w%** |
|---|---|---|
| 1 | Ethanol (200 proof) | 64.87 |
| 2 | Peroxydone K-90 | 31.43 |
| 3 | PEG-4600 | 0.50 |
| 4 | PEG-400 | 3.00 |
| 5 | Menthol | 0.10 |
| 6 | Sodium Saccharin | 0.10 |
| | Total | 100.00 |

The third layer (barrier layer) was cast from the following formulation:

| | **Ingredient** | **w/w %** |
|---|---|---|
| 1 | Ethanol (190 proof) | 69.80 |
| 2 | PEG 1000 | 1.20 |
| 3 | HP-55S | 18.00 |
| 4 | DI Water | 10.00 |
| 5 | Doublemint oil based | 1.00 |
| | Total | 100.00 |

To make the dental strip, the third layer was first cast onto a Teflon substrate. The third layer was allowed to dry overnight in a hood. The second layer was then cast on top of the third layer and allowed to dry. Finally, the adherent layer (first layer) was cast onto the top of the second layer and allowed to dry. During the formation of the strip, water is generally avoided to prevent reversion of the amorphous calcium phosphate to an inactive, insoluble form.

The three layer strip of Example 1 was tested using X-ray diffraction (XRD), and the results indicated that the calcium phosphate in the strip remained amorphous, and had not reverted to an insoluble form. Testing also indicated that the strip released calcium ions. Furthermore, the strip bleached teeth, increased the hardness of tooth chips, and increased mineral volume of tooth chips.

### Example 2: Three Layer Oral Care Strip / ACP and Whitening Agent in a Single Layer

The first layer (the adherent layer) was cast from the following formulation:

| | **Ingredient** | **w/w %** |
|---|---|---|
| 1 | Ethanol (200 proof) | 63.2 |
| 2 | Peroxydone K-90 | 35.00 |
| 3 | PEG-400 | 0.50 |
| 4 | Menthol | 0.15 |
| 5 | Sodium Saccharin | 0.15 |
| 6 | Si-ACP | 1.00 |
| | Total | 100.00 |

The second layer (middle layer) was cast from the following formulation:

| | **Ingredient** | **w/w %** |
|---|---|---|
| 1 | Ethanol (200 proof) | 94.40 |
| 2 | PVP K-90 | 5.50 |
| 3 | Menthol | 0.05 |
| 4 | Sodium Saccharin | 0.05 |
| | Total | 100.00 |

The third layer (barrier layer) was cast from the following formulation:

| | **Ingredients** | **w/w %** |
|---|---|---|
| 1 | Ethanol - 95% (190 proof) | 69.80 |
| 2 | PEG 1000 | 1.20 |
| 3 | HP-55S | 18.00 |
| 4 | DI Water | 10.00 |
| 5 | Flavor Doublemint oil based | 1.00 |
| | Total | 100.00 |

The strip was made by casting according to the procedure described above in Example 1.

### Example 3: A Single Layer Oral Care Strip

The strip was cast from the following formulation.

| | **Ingredient** | **w/w %** |
|---|---|---|
| 1 | Ethanol (200 proof) | 49.50 |
| 2 | Peroxydone K-90 | 30.43 |
| 3 | HP55S | 15.57 |
| 4 | PEG-400 | 2.00 |
| 5 | Menthol | 0.5 |
| 6 | Sodium Saccharin | 0.5 |
| 7 | Si-ACP | 1.50 |
| | Total | 100.00 |

### OTHER EMBODIMENTS

Other embodiments are within the scope of the claims.

While embodiments of devices have been described that are in strip-form, embodiments are possible in which the oral care device is in other forms, e.g., in the form of a mouthpiece, e.g., a heat moldable mouthpiece, that is configured to be worn by a user.

While strips have been shown in which an amorphous calcium phosphate and a whitening agent are in separate layers, in some embodiments, they are in a single layers. The amorphous calcium phosphate and/or whitening agent can be encapsulated, e.g., micro-encapsulated in a protective shell that can be soluble or swollen by water.

While strips have been described that have three layers, other configurations are possible. For example, Fig. 2 shows a strip 30 that includes two layers, while Fig. 3 shows a strip 40 that includes one layer. Still more layers are possible. For example, a strip can contain, e.g., four, five, six, seven or more, e.g., eleven layers. This paragraph also applies to other devices, e.g., mouthpieces.

## Claims

1. An oral care device configured to contact one or more teeth, comprising an amorphous calcium phosphate or a precursor or derivative thereof, and a tooth whitening agent.

2. The oral care device of claim 1, wherein the amorphous calcium phosphate comprises a stabilized amorphous calcium phosphate.

3. The oral care device of claim 2, wherein the stabilized amorphous calcium phosphate comprises a metal-stabilized or metalloid-stabilized calcium phosphate.

4. The oral care device of claim 3, wherein the metal or metalloid is selected from the group consisting of zirconium, silicon, titanium, and mixtures thereof.

5. The oral care device of claim 2, wherein the stabilized amorphous calcium phosphate is selected from the group consisting of zirconium-modified amorphous calcium phosphate (Zr-ACP), titanium-modified amorphous calcium phosphate (Ti-ACP), silicon-modified amorphous calcium phosphate (Si-ACP), amorphous calcium phosphate fluoride (ACPF), amorphous calcium carbonate phosphate (ACCP).

6. The oral care device of claim 2, wherein the stabilized amorphous calcium phosphate comprises an amino acid, a polypeptide or derivatives of an amino acid or a polypeptide.

7. The oral care device of claim 6, wherein the polypeptide comprises a casein phosphopeptide, or a derivative thereof.

8. The oral care device of claim 2, wherein the stabilized amorphous calcium phosphate comprises a synthetic polymer.

9. The oral care device of claim 1, wherein the tooth whitening agent is selected from the group consisting of peroxides, metal chlorites, perborates, percarbonates, peroxyacids, persulfates, carbamides, hypochlorites, chlorine dioxide, and mixtures thereof.

10. The oral care device of claim 1, wherein the tooth whitening agent comprises hydrogen peroxide, a complex of thereof, or a source thereof.

11. The oral care device of claim 1, wherein the device is in the form of a strip.

12. The oral care device of claim 11, wherein the strip comprises a polymeric material.

13. The oral care device of claim 12, wherein the polymeric material is selected from the group consisting polyvinylpyrrolidone, a cellulosic polymer, polyvinyl alcohol, hydroxyethyl cellulose, hydroxypropyl cellulose, hydroxypropylmethyl cellulose, sodium carboxymethyl cellulose, polyethylene oxide, polyethylene glycol, polyacrylic acid, silicone, hydroxypropylmethyl-cellulose phthalate (HPMCP), polyvinyl acetate, cellulose acetate phthalate, gelatin sodium alginate, carbomer, polyvinyl pyrrolidone-vinyl acetate copolymer, polyalkylvinylether-maleic acid copolymer, polypropylene glycol, polyethylene glycol-polypropylene glycol copolymers, quaternized copolymers, quaternized copolymer of vinylpyrrolidone and dimethylaminoethyl methacrylate, and mixtures thereof.

14. The oral care device of claim 12, wherein the polymeric material is soluble or swollen by water.

15. The oral care device of claim 12, wherein the polymeric has a flexural modulus less than 100,000 psi, as measured using a dynamic mechanical analyzer.

16. The oral care device of claim 15, wherein the flexural modulus is less than 25,000 psi.

17. The oral care device of claim 12, wherein the polymeric material has a hardness of less than 100 Shore A.

18. The oral care device of claim 17, wherein the hardness is less than 50 Shore A.

19. The oral care device of claim 11, wherein the strip has more than a single layer.

20. The oral care device of claim 11, wherein the strip has two layers.

21. The oral care device of claim 11, wherein the strip has three layers.

22. The oral care device of claim 11, wherein the strip includes two layers, and wherein each layer is formed of a different polymeric material.

23. The oral care device of claim 11, wherein the strip includes:
a first layer configured to contact one or more teeth that comprises the amorphous calcium phosphate;
a third layer; and
a second layer sandwiched between the first and third layers, the second layer comprising the whitening agent.

24. The oral care device of claim 23, wherein the first layer comprises polyvinylpyrrolidone, the second layer comprises polyvinylpyrrolidone, and the third layer comprises hydroxypropylmethylcellulose phthalate (HPMCP).

25. The oral care device of claim 23, wherein the molecular weight of the HPMCP is greater than 100,000, as measured relative to mono-disperse polyethylene glycol standards in tetrahydrofuran as solvent.

26. The oral care device of claim 23, wherein an overall thickness of the strip is from about 0.1 mm to about 2.5 mm.

27. The oral care device of claim 23, wherein a thickness of the first layer is from about 0.001 mm to about 0.5 mm.

28. The oral care device of claim 23, wherein a thickness of the second layer is from about 0.05 mm to about 1.0 mm.

29. The oral care device of claim 23, wherein a thickness of the third layer is from about 0.05 mm to about 0.5 mm.

30. The oral care device of claim 23, wherein the layers are formed by casting.

31. A method of treating teeth, the method comprising:
contacting one or more teeth with a device comprising an amorphous calcium phosphate or a precursor or derivative thereof, and a tooth whitening agent; and
maintaining contact with the one or more teeth for a sufficient time to at least whiten the one or more teeth.

32. An oral care composition, comprising an amorphous calcium phosphate or a precursor or derivative thereof; and a tooth whitening agent.

33. The oral care composition of claim 32, wherein the amorphous calcium phosphate comprises a stabilized calcium phosphate.

34. The oral care composition of claim 33, wherein the stabilized amorphous calcium phosphate comprises a metal-stabilized or metalloid-stabilized calcium phosphate.

35. The oral care composition of claim 34, wherein the metal or metalloid is selected from the group consisting of zirconium, silicon, titanium, and mixtures thereof.

36. The oral care composition of claim 33, wherein the stabilized amorphous calcium phosphate is selected from the group consisting of zirconium-modified amorphous calcium phosphate (Zr-ACP), titanium-modified amorphous calcium phosphate (Ti-ACP), silicon-modified amorphous calcium phosphate (Si-ACP), amorphous calcium phosphate fluoride (ACPF), amorphous calcium carbonate phosphate (ACCP).

37. The oral care composition of claim 33, wherein the stabilized amorphous calcium phosphate comprises a polypeptide.

38. The oral care composition of claim 37, wherein the polypeptide comprises a casein phosphopeptide, or a derivative thereof.

39. The oral care composition of claim 33, wherein the stabilized amorphous calcium phosphate comprises a synthetic polymer.

40. The oral care composition of claim 32, wherein the tooth whitening agent is selected from the group consisting of peroxides, metal chlorites, perborates, percarbonates, peroxyacids, persulfates, carbamides, hypochlorites, chlorine dioxide, and mixtures thereof.

41. The oral care composition of claim 32, wherein the tooth whitening agent comprises hydrogen peroxide, a complex of thereof,or a source thereof.

42. The oral care composition of claim 41, wherein the composition comprises from about 0.1 to about 20% hydrogen peroxide.

43. The oral care composition of claim 32, further comprising a polymeric material.

44. The oral care composition of claim 32, further comprising a solvent.

45. The oral care composition of claim 44, wherein the solvent is a volatile solvent, having a normal atmospheric boiling point of less than 100 °C.

46. The oral care composition of claim 44, wherein the solvent is selected from the group consisting of ethanol, polyols, glycols, glycerol, propylene glycol, and mixtures thereof.

47. The oral care composition of claim 44, wherein the solvent is substantially free of water.

48. The oral care composition of claim of claim 44, wherein the solvent includes less than 0.1 percent by weight water.

49. The composition of claim 32, further comprising a sweetener, a flavoring or a fragrance.

50. The oral care composition of claim 32, wherein the composition is in the form of a liquid.

51. The oral care composition of claim 32, wherein the composition is in the form of a gel.

52. The oral care composition of claim 32, wherein the composition is in the form of a dentifrice.

53. The oral care composition of claim 32, wherein the composition is in the form of a rinse.

54. The oral care composition of claim 32, wherein the composition is in the form of a varnish or paint.

55. A method of treating teeth, the method comprising
applying to a tooth, an oral care composition comprising an amorphous calcium phosphate or a precursor or derivative thereof and a whitening agent; and
allowing the oral care composition to maintain contact with the tooth for sufficient time to at least whiten the tooth.

56. The method of claim 55, wherein the sufficient time is less than 5 minutes.

57. The method of claim 55, wherein the sufficient time is less than 10 minutes.

58. The method of claim 55, wherein the sufficient time is less than 30 minutes.

59. A method of simultaneously whitening and remineralizing teeth, the method comprising:
contacting one or more teeth with a device that includes an amorphous calcium phosphate or a precursor or derivative thereof, and a tooth whitening agent; and
maintaining contact with the one or more teeth for a sufficient time to at least whiten the one or more teeth.

60. The method of claim 59, wherein the method also at least partially remineralizes the one or more teeth.

61. The method of claim 59, wherein the method also reduces sensitivity of the one or more teeth.
